# EUROPEAN PATENT APPLICATION

(11) **EP 4 295 890 A1**
(43) Date of publication of application: **27.12.2023**
(21) Application number: 21926727.5
(22) Date of filing: 22.11.2021
(51) Int. Cl.: A61M 25/10

(54) **BALLOON CATHETER**

(30) Priority: 17.02.2021 JP 2021023339
(71) Applicant: Asahi Intecc Co., Ltd., Seto-shi, Aichi 489-0071 (JP)
(72) Inventor: ENDOU, Syouta, Seto-shi, Aichi 489-0071 (JP)
(74) Representative: Prinz & Partner mbB
(86) International application number: PCT/JP2021/042771
(87) International publication number: WO 2022/176299

(57) **Abstract**

An object is to provide a balloon catheter that allows a balloon to rapidly contract even if the balloon located right above an opening is strongly pressed against an outer peripheral surface of a hollow shaft when contracting the balloon.

A balloon catheter 1 includes a hollow shaft 11 having a lumen 11h1 through which an expansion liquid flows, and an expandable and contractable balloon 31 arranged so as to cover at least a part of an outer periphery of the hollow shaft 11. The hollow shaft 11 has an opening 11a1 that communicates between the lumen 11h1 and an inside of the balloon 31, and an outer peripheral surface 11b of the hollow shaft 11 has a groove 111 extending across the opening 11a1 and along the outer peripheral surface 11b.

## Description

### TECHNICAL FIELD

The disclosed embodiments relate to a balloon catheter.

### BACKGROUND ART

For example, balloon catheters are known as medical instruments for dilating a constricted part in a blood vessel. A balloon catheter is generally composed of a balloon for dilating blood vessels and a hollow shaft for delivering an expansion liquid into the balloon.

In a procedure using such a balloon catheter, after completion of dilation of the constricted part, the balloon is contracted by draining the expansion liquid in the balloon through an opening provided on the hollow shaft, and the balloon catheter is removed out of the body (e.g. see Patent Literature 1).

### CITATION LIST

### Patent Literature

Patent Literature 1: JP 2016-116814 A

### SUMMARY OF INVENTION

### TECHNICAL PROBLEM

However, in the procedure using the conventional balloon catheter as described above, the opening on the hollow shaft may be occluded by the balloon when contracting the balloon. Such occlusion of the opening can occur e.g. when the hollow shaft on a site corresponding to the balloon is pressed against a wall of a blood vessel and the balloon located right above the opening can be strongly pressed against the outer peripheral surface of the hollow shaft. In such a case, there is a possibility that the expansion liquid is insufficiently drained due to occlusion of the opening by the balloon, and the balloon catheter is prevented from being smoothly removed e.g. because the balloon does not contract as intended.

The disclosed embodiments have been made in view of the circumstances described above, and an object of the disclosed embodiments is to provide a balloon catheter that allows a balloon to rapidly contract even if the balloon located above an opening is strongly pressed against an outer peripheral surface of a hollow shaft when contracting the balloon.

### SOLUTION TO PROBLEM

The present disclosure includes some aspects described below.
(1) A balloon catheter including a hollow shaft having a lumen through which an expansion liquid flows, and an expandable and contractable balloon arranged so as to cover at least a part of an outer periphery of the hollow shaft, in which the hollow shaft has an opening that communicates between the lumen and the inside of the balloon, and an outer peripheral surface of the hollow shaft has a groove extending across the opening and along the outer peripheral surface.
(2) The balloon catheter according to (1), in which the groove extends along a circumferential direction of the hollow shaft.
(3) The balloon catheter according to (2), in which the groove annularly extends over the entire circumferential direction of the hollow shaft.
(4) The balloon catheter according to (1), in which the groove extends along a direction inclined relative to a longitudinal direction of the hollow shaft.
(5) The balloon catheter according to any one of (1) to (4), in which the groove includes a plurality of grooves on one opening.

### ADVANTAGEOUS EFFECTS OF INVENTION

The disclosed embodiments can provide a balloon catheter that allows a balloon to rapidly contract even if the balloon located above an opening is strongly pressed against an outer peripheral surface of a hollow shaft when contracting the balloon.

### BRIEF DESCRIPTION OF DRAWINGS

FIG. 1A is a schematic plane view illustrating a whole of the first embodiment.
FIG. 1B is a schematic side view illustrating a whole of the first embodiment.
FIG. 2A is a schematic enlarged plane view illustrating a part of FIG. 1A.
FIG. 2B is a schematic enlarged side view illustrating a part of FIG. 1B.
FIG. 3 is a schematic sectional view taken along line III-III in FIG. 2B.
FIG. 4 is a schematic enlarged side view illustrating a part of the second embodiment.
FIG. 5 is a schematic sectional view taken along line V-V in FIG. 4.
FIG. 6 is a schematic enlarged side view illustrating a part of the third embodiment.
FIG. 7A is a schematic enlarged plane view illustrating a part of the fourth embodiment.
FIG. 7B is a schematic enlarged side view illustrating a part of the fourth embodiment.
FIG. 8A is a schematic enlarged side view illustrating a part of another embodiment.
FIG. 8B is a schematic enlarged side view illustrating a part of another embodiment.
FIG. 8C is a schematic sectional view illustrating another embodiment.
FIG. 8D is a schematic sectional view illustrating another embodiment.
FIG. 8E is a schematic enlarged side view illustrating a part of another embodiment.
FIG. 8F is a schematic enlarged side view illustrating a part of another embodiment.

### DESCRIPTION OF EMBODIMENTS

A balloon catheter according to the present disclosure include a hollow shaft having a lumen through which an expansion liquid flows, and an expandable and contractable balloon arranged so as to cover at least a part of an outer periphery of the hollow shaft, in which the hollow shaft has an opening that communicates between the lumen and the inside of the balloon, and an outer peripheral surface of the hollow shaft has a groove extending across the opening and along the outer peripheral surface.

In the present specification, a "distal end side" means a direction along an axis direction of the hollow shaft, which is an advancing direction of the balloon catheter toward a treatment site. A "proximal end side" means a direction along the axis direction of the hollow shaft and opposite to the distal end side. Further, a "distal end" means an end portion on a distal end side of any member or portion, and a "proximal end" means an end portion on a proximal end side of any member or portion.

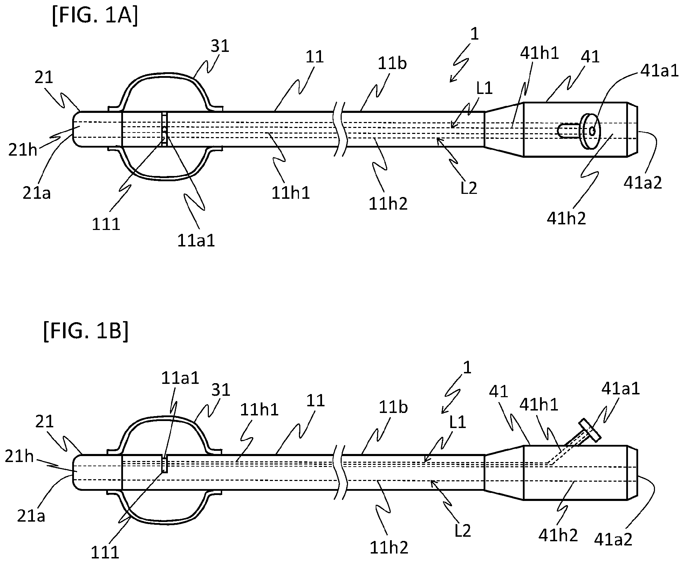

Hereinafter, the first to fourth embodiments will be described with reference to the figures, however the disclosed embodiments are not limited only to the embodiments below. Furthermore, the dimensions of the balloon catheter illustrated in each figure are indicated to facilitate the understanding of the contents of the implementation and do not correspond to the actual dimensions. Also, in each figure, the left side of the figure is the distal end side (farther side) to be inserted into a body, and the right side is the proximal end side (nearer side) to be operated by an operator such as a surgeon. In the figures other than FIG. 1A and FIG. 1B, the balloon is omitted.

### [First Embodiment]

FIG. 1 to FIG. 3 are schematic diagrams illustrating the first embodiment. As illustrated in FIG. 1A and FIG. 1B, a balloon catheter 1 is schematically composed of a hollow shaft 11, a distal tip 21, a balloon 31, and a connector 41.

The hollow shaft 11 has a lumen through which the expansion liquid flows. The hollow shaft 11 has, on its distal end portion, an opening 11a1 that communicates between a lumen 11h1 of the hollow shaft and an inside of the balloon described later. The lumen 11h1 is arranged so as to penetrate the hollow shaft 11 from the opening 11a1 to the proximal end portion. The balloon catheter 1 according to the first embodiment has a lumen 11h2 in addition to the lumen 11h1. The lumen 11h2 is arranged so as to be parallel to the lumen 11h1 and to penetrate the hollow shaft 11 from the distal end to the proximal end.

An outer peripheral surface 11b of the hollow shaft 11 has a groove 111 extending across the opening 11a1 and along the outer peripheral surface 11b. The groove 111 is concave toward a central axis direction from the outer peripheral surface 1 1b of the hollow shaft. The extending direction of the groove 111 is not particularly limited. Examples of the extending direction include a circumferential direction of the hollow shaft 11, a longitudinal direction of the hollow shaft 11, a direction inclined relative to the longitudinal direction of the hollow shaft 11 (including a spiral direction), and a combination thereof. The groove 111 may extend in one direction or in multiple directions starting from the opening 11a1.

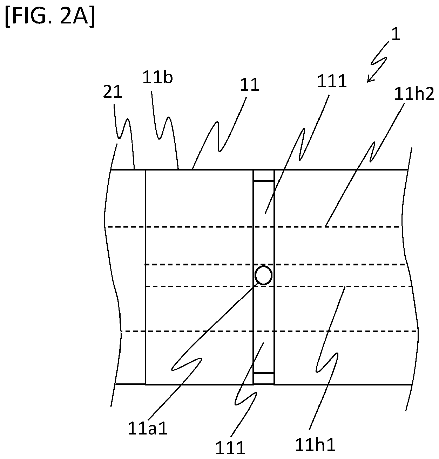

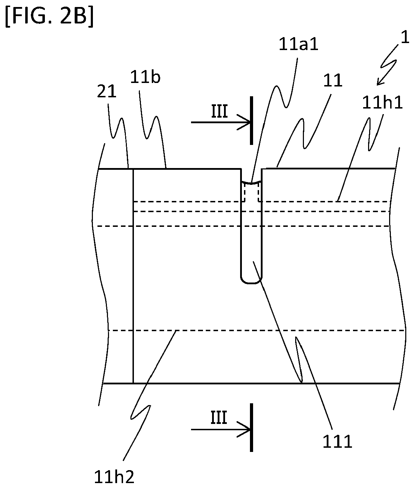

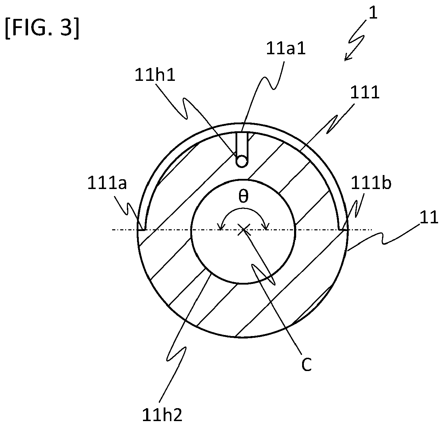

When the groove 111 is formed in a direction inclined relative to the circumferential direction or the longitudinal direction of the hollow shaft 11, an angle between both longitudinal end portions 111a/111b of the groove 111 and a central axis C of the hollow shaft 11 (angle θ in FIG. 3) is not particularly limited unless the effects of the disclosed embodiments are impaired. For example, the angle may be 90° or 180°. In the first embodiment, FIG. 2A, FIG. 2B, and FIG. 3 illustrate a configuration in which the groove 111 extends in two opposing directions starting from the opening 11a1, and an angle between the both end portions of the groove 111 and the central axis is 180°.

Herein, a width of the groove 111 may be the same as a diameter of the opening 11a1, but may be smaller or larger than the diameter of the opening. If the width of the groove 111 is smaller than the diameter of the opening, for example, the balloon 31 described later hardly occlude the groove 111, and the flow passage for the expansion liquid can be more secured. If the width of the groove 111 is greater than the diameter of the opening, for example, the flow rate in the groove 111 can be increased, so that the balloon 31 can be more rapidly contracted.

The distal tip 21 is a hollow member connected to the distal end of the hollow shaft 11. Specifically, the distal tip 21 includes e.g. a lumen 21h that penetrates the distal tip along the longitudinal direction, and can be formed such that its distal end portion has an approximate sharp shape toward the distal end side. When the balloon catheter 1 includes the distal tip 21, for example, a resistance caused by advance of the balloon catheter in the blood vessel can be decreased, so that the balloon catheter 1 can advance smoothly.

The balloon 31 is an expandable and contractable member arranged so as to cover at least a part of the outer periphery of the hollow shaft. Specifically, for example, the balloon 31 can be arranged so as to cover the outer peripheral surface 11b on the distal end portion of the hollow shaft 11 having the opening 11a1. The distal end of the balloon 31 can be connected to the distal end portion of the hollow shaft 11 and/or the distal tip 21 (any site of the distal tip 21, e.g. a distal end portion, a proximal end portion, etc. of the distal tip 21), and the proximal end portion of the balloon 31 can be connected to a midway of the hollow shaft 11 in the longitudinal direction.

The balloon 31 expands (the diameter increases radially outward) by injecting an expansion liquid into the balloon through the opening 11a1, and contracts (the diameter decreases radially inward) by draining the expansion liquid through the opening 11a1. Thereby, for example, an inner wall of a blood vessel can be pushed and widened, or a stent can be expanded and placed in the blood vessel.

It is preferable that materials constituting the hollow shaft 11, the distal tip 21, and the balloon 31 described above have antithrombogenicity, flexibility, and biocompatibility, because these members are inserted into a body cavity. As the materials mentioned above, for example, resin materials such as polyamides, polyamide elastomers, polyolefins, polyesters, polyester elastomers, polyurethanes, silicones, and fluororesins, can be used.

Examples of adoptable methods of connecting the hollow shaft 11, the distal tip 21, and the balloon 31 to each other include a method of welding resin materials together by heating, a method of bonding resin materials using an adhesive, and the like.

The connector 41 is a member for an operator to grip the proximal end portion of the balloon catheter 1. The connector 41 has a lumen 41h1 communicating with the lumen 11h1, and a lumen 41h2 communicating with the lumen 11h2. The connector 41 can be connected to the proximal end portion of the hollow shaft 11. The configuration of the connector 41 is not particularly limited unless the effects of the disclosed embodiments are impaired.

Herein, the lumen 11h1 of the hollow shaft 11 and the lumen 41h1 of the connector 41 communicate with each other to form an expansion lumen L1 having the opening 11a1 and a proximal opening 41a1. The expansion lumen L1 is a lumen through which the expansion liquid flows. For example, an indeflator (not illustrated) that controls the feeding of the expansion liquid such as physiological saline is connected to the proximal end portion (opening 41a1) of the expansion lumen L1. The lumen 21h of the distal tip 21, the lumen 11h2 of the hollow shaft 11, and the lumen 41h2 of the connector 41 are connected to each other to form a guide wire lumen L2 having a distal opening 21a and a proximal opening 41a2. For example, a guide wire or the like that guides the balloon catheter 1 along the blood vessel is inserted through the guide wire lumen L2.

Next, a usage mode for the balloon catheter 1 will be explained. Herein, a procedure for dilating a constricted part (treatment site) in a coronary artery of a heart using the balloon catheter 1 will be explained.

First, a guide wire (not illustrated) is pushed forward to a treatment site prior to insertion of the balloon catheter 1. Next, while the balloon 31 of the balloon catheter 1 is decreased in diameter, the proximal end of the guide wire is inserted into the opening 21a of the guide wire lumen L2 of the balloon catheter 1, and the balloon catheter 1 is inserted, from its distal end, into the blood vessel.

Next, the balloon catheter 1 is pushed forward along the guide wire to the treatment site. Subsequently, the balloon catheter 1 is placed in the blood vessel while the balloon 31 reaches the inside of the constricted part, and then the expansion liquid such as physiological saline is injected into the balloon 31 from the opening 41a1 through the expansion lumen L1 to expand the balloon 31. In this process, the constricted part is dilated by pushing and widening the inner wall of the constricted part while the outer peripheral surface of the expanded balloon 31 is in contact with the inner wall of the constricted part.

Next, after completion of the dilation of the constricted part, the expansion liquid is drained from the balloon 31 through the opening 11a1 while suctioning the expansion liquid using an indeflator, to decrease the balloon 31 in diameter. In this process, some of the expansion liquid in the balloon 31 once enters the groove 111, then reaches the opening 11a1 along the groove 111, and is sucked into lumen 11h1 from the opening 11a1. Then, after the balloon 31 is contracted, the balloon catheter 1 is removed out of the body while retracting the balloon catheter 1 to complete the procedure for the balloon catheter 1.

As described above, the aforementioned configuration of the balloon catheter 1 makes it possible to drain the expansion liquid in the balloon 31 to the lumen 11h1 through the groove 111 to rapidly contract the balloon 31, even if the balloon 31 located right above the opening 11a1 is strongly pressed against the outer peripheral surface 11b of the hollow shaft 11 when contracting the balloon 31.

Since the groove 111 extends along the circumferential direction of the hollow shaft 11 in the balloon catheter 1, for example, even if a certain direction in the circumferential direction of the hollow shaft 11 is pressed against the blood vessel wall, the expansion liquid can be drained from the groove 111 extending in a direction other than the certain direction. Thereby, the risk of complete occlusion of the groove 111 (flow passage through which the expansion liquid is drained) due to the balloon 31 can be more reduced.

### [Second Embodiment]

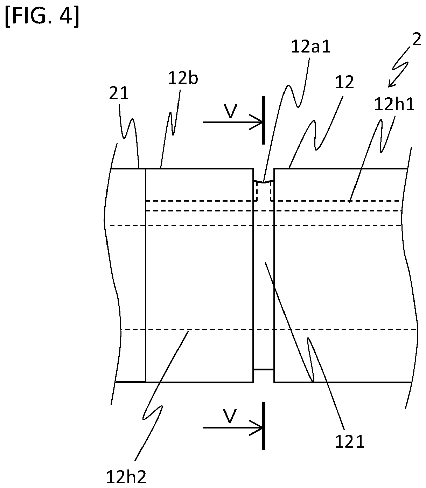

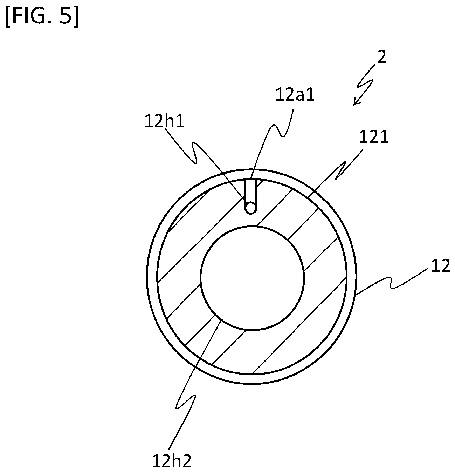

FIG. 4 and FIG. 5 are schematic diagrams illustrating the second embodiment. As illustrated in FIG. 4, a balloon catheter 2 is schematically composed of the hollow shaft 12, the distal tip 21, the balloon 31 (not illustrated), and the connector 41 (not illustrated). In the balloon catheter 2 according to the second embodiment, the hollow shaft 12 has a configuration different from that of the hollow shaft in the first embodiment. Since the configurations of the distal tip 21, the balloon 31, and the connector 41 are the same as those in the first embodiment, the same parts are designated by the same reference numerals and detailed description thereof will be omitted. The second embodiment is configured in the same manner as in the first embodiment except for the configuration of the hollow shaft 12 described below.

The hollow shaft 12 has a lumen through which the expansion liquid flows. The hollow shaft 12 has, on its distal end portion, an opening 12a1 that communicates between a lumen 12h1 of the hollow shaft 12 and the inside of the balloon 31. An outer peripheral surface 12b of the hollow shaft 12 has a groove 121 extending across the opening 12a1 and along the outer peripheral surface 12b.

The groove 121 according to the second embodiment extends annularly over the entire circumferential direction of the hollow shaft 12. Specifically, the groove 121 extends to e.g. a site opposite to the opening 12a1 in the circumferential direction of hollow shaft 12, so that the expansion liquid can flow in any direction of the circumferential direction to proceed toward the opening 12a1.

As described above, the aforementioned configuration of the balloon catheter 2 makes it possible to secure a flow passage through which the expansion liquid is drained without occlusion of the groove 121 due to the balloon 31 on at least a site opposite to a site of the hollow shaft 12 pressed against the blood vessel wall. Thereby, the risk of complete occlusion of the groove 121 (flow passage through which the expansion liquid is drained) due to the balloon 31 can be more reduced.

### [Third Embodiment]

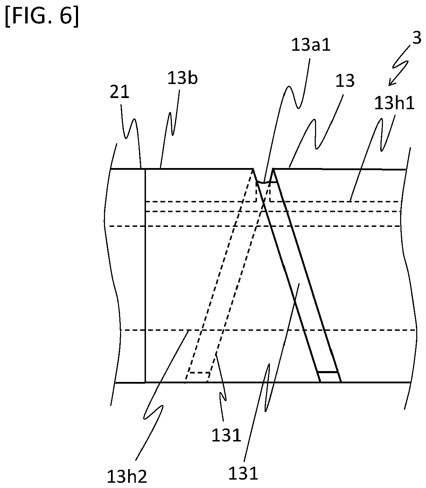

FIG. 6 is a schematic diagram illustrating the third embodiment. As illustrated in FIG. 6, a balloon catheter 3 is schematically composed of a hollow shaft 13, the distal tip 21, the balloon 31 (not illustrated), and the connector 41 (not illustrated). In the balloon catheter 3 according to the third embodiment, the hollow shaft 13 has a configuration different from that of the hollow shaft in the first embodiment. Since the configurations of the distal tip 21, the balloon 31, and the connector 41 are the same as those in the first embodiment, the same parts are designated by the same reference numerals and detailed description thereof will be omitted. The third embodiment is configured in the same manner as in the first embodiment except for the configuration of the hollow shaft 13 described below.

The hollow shaft 13 has a lumen through which the expansion liquid flows. The hollow shaft 13 has, on its distal end portion, an opening 13a1 that communicates between a lumen 13h1 of the hollow shaft 13 and the inside of the balloon 31. An outer peripheral surface 13b of the hollow shaft 13 has a groove 131 extending across the opening 13a1 and along the outer peripheral surface 13b.

The groove 131 according to the third embodiment extends along a direction inclined relative to the longitudinal direction of the hollow shaft 13. The third embodiment illustrates a configuration in which the groove 131 spirally extends in two opposing directions starting from the opening 13a1, and an angle between the both end portions of the groove 131 and the central axis of the hollow shaft 13 is 360°.

As described above, the aforementioned configuration of the balloon catheter 3 makes it possible to more reduce the risk of complete occlusion of the groove 131 (flow passage through which the expansion liquid is drained) due to the balloon 31 because the groove 131 also extends in the longitudinal direction.

### [Fourth Embodiment]

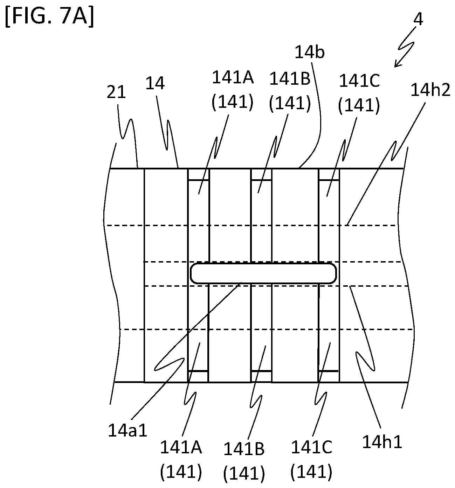

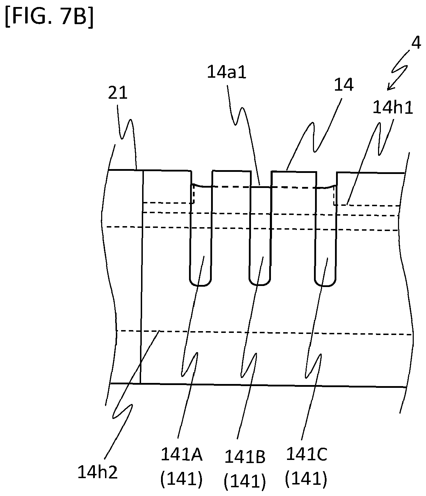

FIG. 7 is a schematic diagram illustrating the fourth embodiment. As illustrated in FIG. 7A and FIG. 7B, a balloon catheter 4 is schematically composed of a hollow shaft 14, the distal tip 21, the balloon 31 (not illustrated), and the connector 41 (not illustrated). In the balloon catheter 4 according to the fourth embodiment, the hollow shaft 14 has a configuration different from that of the hollow shaft in the first embodiment. Since the configurations of the distal tip 21, the balloon 31, and the connector 41 are the same as those in the first embodiment, the same parts are designated by the same reference numerals and detailed description thereof will be omitted. The fourth embodiment is configured in the same manner as in the first embodiment except for the configuration of the hollow shaft 14 described below.

The hollow shaft 14 has a lumen through which the expansion liquid flows. The hollow shaft 14 has, on its distal end portion, an opening 14a1 that communicates between a lumen 14h1 of the hollow shaft 14 and the inside of the balloon 31. An outer peripheral surface 14b of the hollow shaft 14 has grooves 141 extending across the opening 14a1 and along the outer peripheral surface 14b.

In the fourth embodiment, a plurality of grooves 141 are provided on one opening. Specifically, in the fourth embodiment, the longitudinal shaped opening 14a1 is provided along the longitudinal direction. In the fourth embodiment, a first groove 141A, a second groove 141B, and a third groove 141C that extend in a semicircular pattern along the circumferential direction are independently arranged in this order from the distal end side to the proximal end side.

As described above, the aforementioned configuration of the balloon catheter 4 makes it possible to more reduce the risk of complete occlusion of the grooves 141 (flow passage through which the expansion liquid is drained) due to the balloon 31 because the grooves 141 are distributed on a plurality of positions.

It should be noted that the present disclosure is not limited to the configurations of the aforementioned embodiments but defined by the scope of claims. The present disclosure is intended to include all modifications within the meaning and scope equivalent to those of claims. A part of the configurations of the aforementioned embodiments may be deleted or replaced with another configuration, and other configurations may be added to the configurations of the aforementioned embodiments.

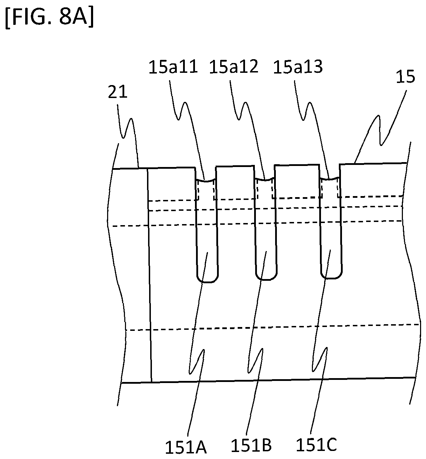

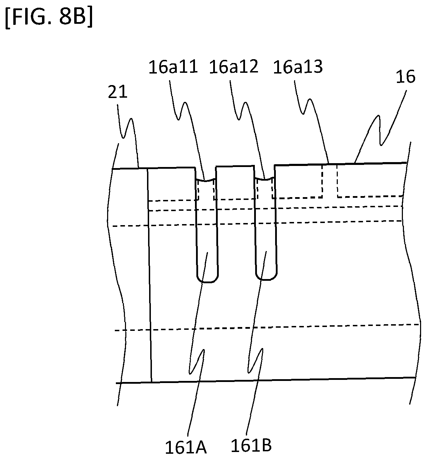

For example, the aforementioned embodiments describe the balloon catheters 1, 2, 3, and 4 including the hollow shafts 11, 12, 13, and 14 respectively, which have one opening 11a1, 12a1, 13a1, and 14a1 respectively. However, the balloon catheter may have a plurality of openings (e.g. 15a11, 15a12, 15a13). In this configuration, each opening may have one or a plurality of grooves (e.g. 151A, 151B, 151C) (see FIG. 8A), or only some of a plurality of openings (e.g. 16a11, 16a12, 16a13) may have grooves (e.g. 161A, 161B) (see FIG. 8B).

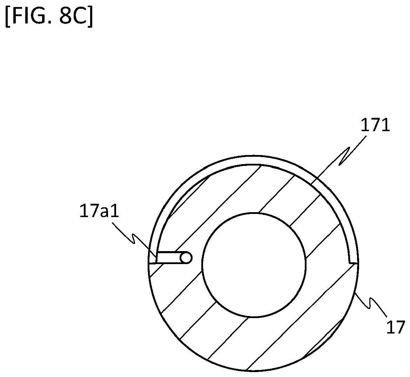

The aforementioned embodiments describe balloon catheters 1, 2, 3, and 4, having the openings 11a1, 12a1, 13a1, and 14a1 respectively located in the midway of the grooves 111, 121, 131, and 141 respectively. However, the balloon catheter may have an opening 17a1 located on an end of a groove 171 (see FIG. 8C).

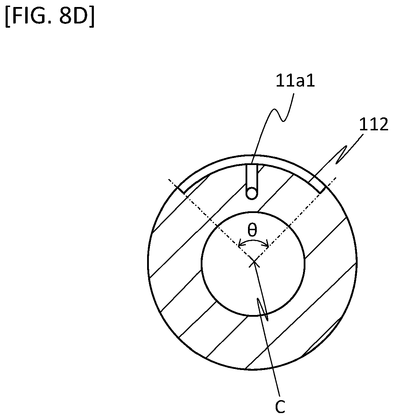

The first embodiment describes the balloon catheter 1 with the groove 111 extending in the semicircular pattern (angle θ=180°). However, in the balloon catheter, the groove 112 may have an angle (e.g. angle θ=90°) other than the angle of the semicircular pattern (angle θ=180°) (see FIG. 8D).

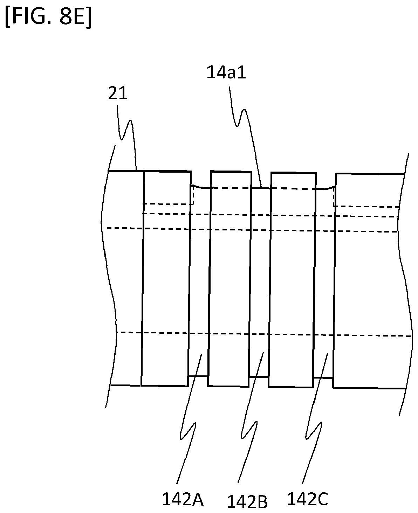

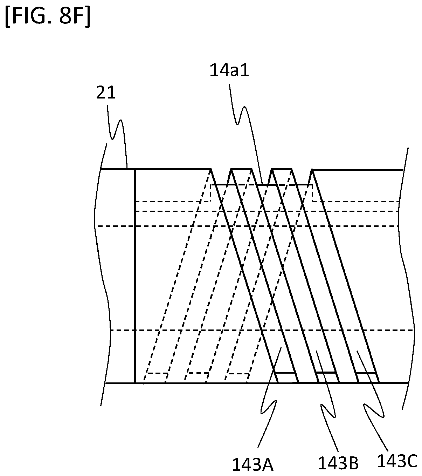

The fourth embodiment describes the balloon catheter 4 that has the plurality of grooves 141 provided on one opening 14a1 and extending in the semicircular pattern (angle θ=180°). However, the balloon catheter may have the plurality of grooves 142A, 142B, and 142C provided on one opening 14a1 and extending annularly over the entire circumferential direction of the hollow shaft (see FIG. 8E). Furthermore, the balloon catheter may have the plurality of grooves 143A, 143B, and 143C provided on one opening 14a1 and extending along a direction inclined relative to the longitudinal direction of the hollow shaft (see FIG. 8F).

The aforementioned embodiments describe the balloon catheters 1, 2, 3, and 4, each including the distal tip 21 and the connector 41. However, the balloon catheters need not include at least any of these components.

### REFERENCE SIGNS LIST

1, 2, 3, 4 Balloon catheter
11, 12, 13, 14 Hollow shaft
11a1, 12a1, 13a1, 14a1 Opening
11b, 12b, 13b, 14b Outer peripheral surface
11h1, 12h1, 13h1, 14h1 Lumen
111, 121, 131, 141, 141A, 141B, 141C Groove
21 Distal tip
31 Balloon
41 Connector

## Claims

1. A balloon catheter comprising:
a hollow shaft having a lumen through which an expansion liquid flows, and
an expandable and contractable balloon arranged so as to cover at least a part of an outer periphery of the hollow shaft, wherein
the hollow shaft has an opening that communicates between the lumen and an inside of the balloon, and
an outer peripheral surface of the hollow shaft has a groove extending across the opening and along the outer peripheral surface.

2. The balloon catheter according to claim 1, wherein the groove extends along a circumferential direction of the hollow shaft.

3. The balloon catheter according to claim 2, wherein the groove annularly extends over the entire circumferential direction of the hollow shaft.

4. The balloon catheter according to claim 1, wherein the groove extends along a direction inclined relative to a longitudinal direction of the hollow shaft.

5. The balloon catheter according to any one of claims 1 to 4, wherein the groove includes a plurality of grooves on one opening.
